# EUROPEAN PATENT APPLICATION

(11) **EP 3 459 466 A2**
(43) Date of publication of application: **27.03.2019**
(21) Application number: 18196485.9
(22) Date of filing: 25.09.2018
(51) Int. Cl.: A61B 10/02

(54) **ANCHOR NEEDLE**

(30) Priority: 26.09.2017 US 201762563431 P
(71) Applicant: Cook Medical Technologies LLC, Bloomington, IN 47404 (US)
(72) Inventor: O'CALLAGHAN, Rory, Shercock County Cavan (IE); SOMERS, Clodagh, Parteen County Clare (IE); CLANCY, Michael S., Limerick (IE)
(74) Representative: Simpson, Tobias Rutger

(57) **Abstract**

Anchor needles and methods of anchoring an anchor needle to target tissue are provided. An anchor needle may be adapted to be arranged in a tissue-anchoring configuration in which: an anchor cannula lock is arranged in a locked anchor cannula configuration that prevents an anchor cannula from longitudinally translating relative to a cannula support; and an outer cannula is longitudinally positioned relative to the anchor cannula so that the anchor cannula allows tissue to be drawn through the transverse slot by the vacuum. A method may include, while applying a vacuum to an anchor cannula lumen adapted to transmit the vacuum to a distal end of the anchor cannula, longitudinally translating an outer cannula relative to an anchor cannula from a blocking configuration to an exposing configuration in which the anchor cannula allows target tissue to be drawn through a transverse slot in the outer cannula by the vacuum.

## Description

### TECHNICAL FIELD

The present disclosure relates generally to medical devices, and more particularly to biopsy needles and biopsy procedures, for example endoscopic ultrasound-guided fine needle aspiration.

### BACKGROUND

Fine needle aspiration is a medical procedure that can be used to retrieve a tissue biopsy sample. Typically, a hollow needle with a sharp, open distal tip is inserted into an area of targeted tissue and a vacuum is applied to a lumen of the needle to aspirate a sample of the targeted tissue into the distal tip of the hollow needle. The needle may be longitudinally translated in a reciprocating manner within the targeted tissue during aspiration. A stylet may be positioned within the lumen of the needle while the needle is being directed to the targeted tissue, for example so that unwanted material does not enter the needle before the distal tip is inserted into the targeted tissue. Some needles for fine needle aspiration may also include one or more transverse openings (also known as apertures, ports, etc.) extending through the walls of the needle and connecting with the lumen. These openings are located proximal to the needle's open distal end, and may be included to provide additional tissue collection. The stylet may also prevent unwanted material from entering these openings before the distal tip has been inserted into the targeted tissue.

Although many different variations of each of the above components and procedural steps have been introduced into the art, there exists a need for designs that provide physicians and other users with more effective, more efficient, and more user-friendly tissue sample collection.

### BRIEF SUMMARY

In typical fine needle aspiration biopsy sampling, the needle's motion through target tissue (e.g. during longitudinal translation to collect a sample in a target lesion) can push the target tissue away from the needle in a manner that makes it difficult to obtain a sample. If an inadequate sample is achieved on the initial pass, the user may need to carry out subsequent passes of the needle, which can increase the procedure time and make the procedure more traumatic for the patient, for example by requiring that the user attempt to collect tissue from additional tissue areas (e.g. by advancing and withdrawing the needle further into and out of a lesion). Accordingly, a need exists for biopsy needles that can anchor target tissue to themselves.

Additionally, because typical fine needle aspiration biopsy needles include a stylet positioned through their lumen when the needle is being guided to the target tissue, and that stylet may need to be withdrawn from the needle in order to obtain a sample, typical fine needle aspiration biopsy procedures often require the assistance of an additional user (e.g. a nurse) to withdraw the stylet from the needle. Moreover, the mere need to withdraw the stylet from the needle creates a risk of the stylet being dropped or bent, which further complicates the procedure. Accordingly, a need exists for biopsy needles that do not require positioning a stylet through the needle lumen for insertion of the needle to the target tissue and then withdrawing the stylet from the needle for sample collection.

Further, in typical fine needle aspiration biopsy procedures the sample is generally retained within the lumen of the needle. To retrieve the sample, the needle's tip is typically exposed and held into a collection container so the sample can be pushed out into the container. However, the needle's distal tip is sharp so that it can penetrate and cut tissue, and thus exposing that distal tip and maneuvering it into a jar presents a needle stick injury. Accordingly, a need exists for biopsy needles in which the sharp edges of the needle can remain sheathed during tissue retrieval to reduce needle stick injuries.

Aspects and embodiments of the present disclosure are configured to address those needs, and in doing so they may address one or more of the above problems and provide various benefits. Aspects and embodiments of the present disclosure may provide biopsy needles that can anchor tissue to themselves in order to increase the likelihood that, and/or the efficiency with which, a user will obtain adequate samples compared with typical biopsy needles. Aspects and embodiments of the present disclosure may provide biopsy needles that eliminate the need to include a stylet during needle insertion and then withdraw that stylet for sample collection, which can simplify procedures and allow assistants such as nurses to carry out other tasks. Aspects and embodiments of the present disclosure may provide biopsy needles in which sharp edges of the needle can remain sheathed during tissue retrieval, for example by retaining the sample in a cannula that lacks a sharp distal edge, which may reduce needle stick injuries. Those of skill in the art, having the benefit of the present disclosure, may recognize that aspects and embodiments of the present disclosure solve additional problems, provide additional benefits, and may, within the scope of the present disclosure, be practiced in additional technological environments, including a variety of biopsy devices and other sample collection tools.

In a first aspect of the present disclosure, an anchor needle may be provided. The anchor needle may include an anchor cannula defining an anchor cannula lumen. The anchor cannula may include an anchor cannula distal end and the anchor cannula lumen may be adapted to transmit a vacuum to the anchor cannula distal end. The anchor needle may further include an outer cannula defining an outer cannula lumen adapted to receive the anchor cannula. The outer cannula may further include an outer cannula proximal end, an outer cannula distal end, and a transverse slot disposed between the outer cannula proximal end and the outer cannula distal end. The anchor needle may further include a cannula support adapted to hold the anchor cannula and the outer cannula such that the anchor cannula and the outer cannula are able to independently longitudinally translate relative to the cannula support. The anchor needle may further include an anchor cannula lock adapted to releasably lock longitudinal translation of the anchor cannula relative to the cannula support. The anchor needle may be adapted to be arranged in a tissue-anchoring configuration in which: the anchor cannula lock is arranged in a locked anchor cannula configuration that prevents the anchor cannula from longitudinally translating relative to the cannula support; and the outer cannula is longitudinally positioned relative to the anchor cannula so that the anchor cannula allows tissue to be drawn through the transverse slot by the vacuum.

In a second aspect of the present disclosure, a method to anchor an anchor needle to target tissue may be provided. The method may include longitudinally positioning an outer cannula in a blocking configuration relative to an anchor cannula, wherein the anchor cannula is received within a lumen defined by the outer cannula, and wherein in the blocking configuration, the anchor cannula prevents tissue from passing through a transverse slot of the outer cannula. The method may further include, in the blocking configuration, locking the anchor cannula to a cannula support such that the anchor cannula is prevented from longitudinally translating relative to the cannula support. The method may further include, while applying a vacuum to an anchor cannula lumen adapted to transmit the vacuum to a distal end of the anchor cannula, longitudinally translating the outer cannula relative to the anchor cannula from the blocking configuration to an exposing configuration in which the anchor cannula allows the target tissue to be drawn through the transverse slot by the vacuum.

### BRIEF DESCRIPTION OF THE DRAWINGS

FIG. 1A is a partial perspective view of an anchor needle;
FIG. 1B is a partial longitudinal section view of the anchor needle of FIG. 1A;
FIG. 1C is an exploded view of the anchor needle of FIG. 1A, with hidden lines showing internal details, and omitting a cannula support;
FIG. 1D is a partial perspective view of another anchor needle;
FIGS. 2A-2E are partial longitudinal section views depicting a method of using the anchor needle of FIG. 1A;
FIG. 3 is a partial perspective view of another anchor needle in an open configuration;
FIG. 4A is a partial perspective view of another anchor needle;
FIG. 4B is a partial longitudinal section view of the anchor needle of FIG. 4A;
FIG. 4C is a partial longitudinal section view of an anchor needle that has an open needle tip;
FIG. 5 illustrates the exterior of another anchor needle;
FIG. 5A is a longitudinal sectional view of the anchor needle of FIG. 5;
FIG. 5B is an enlarged longitudinal sectional view of a distal portion of the anchor needle of FIG. 5;
FIG. 5C is an enlarged longitudinal sectional view of an anchor handle of the anchor needle of FIG. 5;
FIG. 5D is an enlarged longitudinal sectional view of a medial portion of the anchor needle of FIG. 5;
FIG. 6 illustrates the exterior of another anchor needle connected to a vacuum source;
FIG. 6A is a longitudinal section view of the anchor needle of FIG. 6;
FIG. 6B is an enlarged longitudinal sectional view of a distal portion of the anchor needle of FIG. 6;
FIG. 6C is an enlarged longitudinal sectional view of proximal portion of the anchor needle of FIG. 6;
FIG. 6D is an elevation view of an isolated cutting handle of the anchor needle of FIG. 6;
FIG. 6E is a plan view of the isolated cutting handle of FIG. 6D from a proximal end;
FIG. 6F is a perspective view of an isolated inner handle of a cannula support of the anchor needle of FIG. 6; and
FIG. 6G is a plan view of the inner handle of FIG. 6F from a proximal end.

### DETAILED DESCRIPTION

Various embodiments are described below with reference to the drawings in which like elements generally are referred to by like numerals. The relationship and functioning of the various elements of the embodiments may better be understood by reference to the following detailed description. However, embodiments are not limited to those illustrated in the drawings. It should be understood that the drawings are not necessarily to scale, and in certain instances details may have been omitted that are not necessary for an understanding of embodiments disclosed herein, such as - for example -conventional fabrication and assembly. However, drawings may be rendered to scale unless specifically disclaimed.

The invention is defined by the claims, may be embodied in many different forms, and should not be construed as limited to the embodiments set forth herein; rather, these embodiments are provided so that this disclosure will be thorough and complete, and will fully convey enabling disclosure to those skilled in the art. As used in this specification and the claims, the singular forms "a," "an," and "the" include plural referents unless the context clearly dictates otherwise. Reference herein to any industry standards (e.g., ASTM, ANSI, IEEE standards) is defined as complying with the currently published standards as of the original filing date of this disclosure concerning the units, measurements, and testing criteria communicated by those standards unless expressly otherwise defined herein.

The terms "proximal" and "distal" are used herein in the common usage sense where they refer respectively to a handle/doctor-end of a device or related object and a tool/patient-end of a device or related object. The terms "about," "substantially," "generally," and other terms of degree, when used with reference to any volume, dimension, proportion, or other quantitative or qualitative value, are intended to communicate a definite and identifiable value within the standard parameters that would be understood by one of skill in the art (equivalent to a medical device engineer with experience in this field), and should be interpreted to include at least any legal equivalents, minor but functionally-insignificant variants, standard manufacturing tolerances, and including at least mathematically significant figures (although not required to be as broad as the largest range thereof). The term "adapted to" and any variant thereof means that the feature(s) described is structurally configured in a manner to perform a particular operation, and each instance thereof requires specific structure that is explicitly described and/or that will be implicitly apparent to one of skill in the art such that "a [structure] adapted to [do X operation]" is not merely a recitation of a generic "means," nor is it merely a statement of intended use, but rather it imposes specific structural limitations upon the feature thus described.

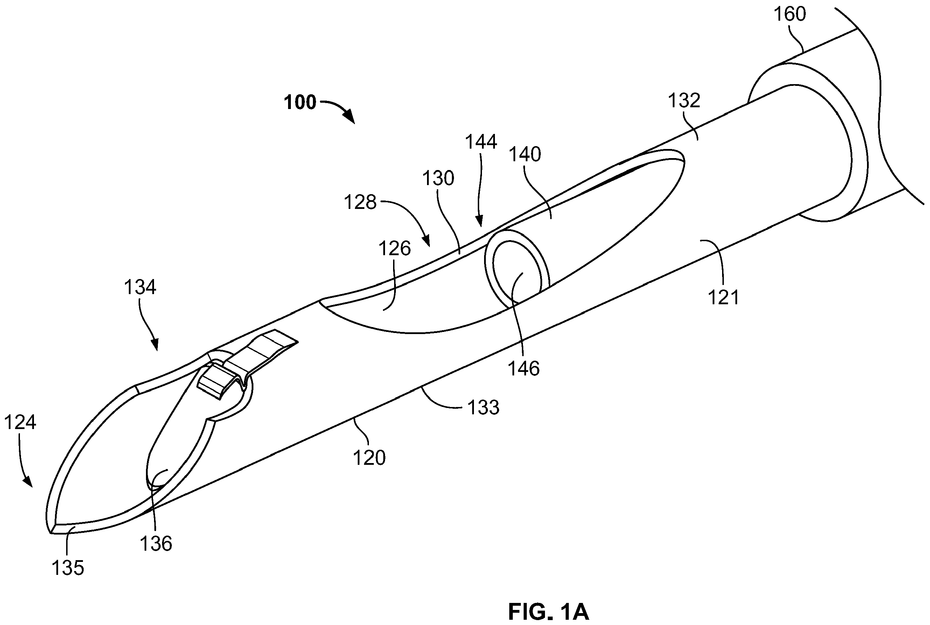

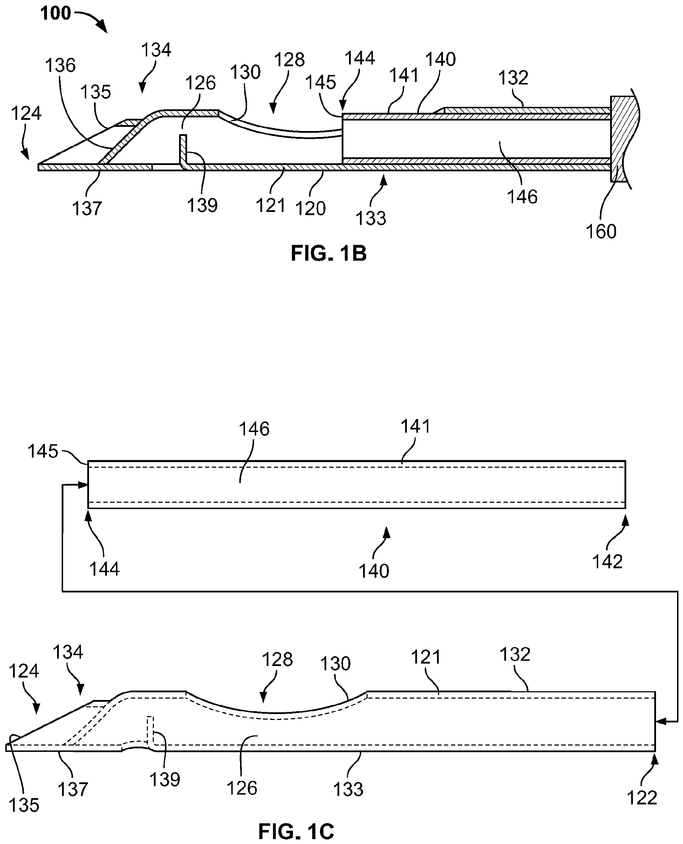

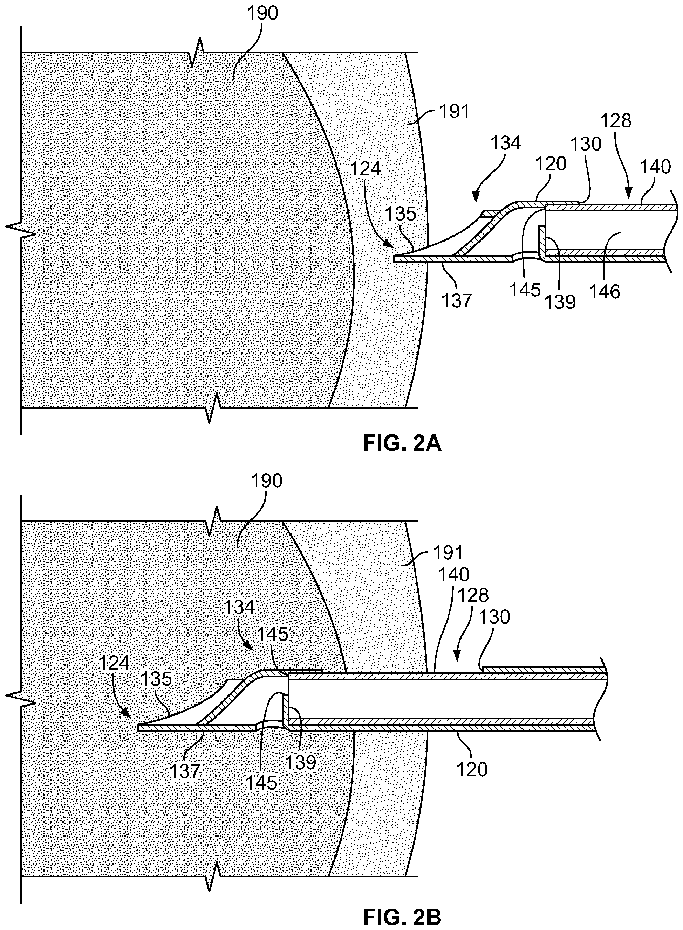

One embodiment of an anchor needle is described with reference to FIGS. 1A-1C and 2A-2E, which illustrate anchor needle 100. Anchor needle 100 is a biopsy needle that includes an outer cannula 120, an anchor cannula 140, and a cannula support 160.

Outer cannula 120 includes a wall 121 that extends from outer cannula proximal end 122 to outer cannula distal end 124. Wall 121 defines an outer cannula lumen 126. Outer cannula lumen 126 is adapted to receive anchor cannula 140 therein, in a generally coaxial manner, such that outer cannula 120 is able to longitudinally translate relative to anchor cannula 140. Put another way, anchor cannula 140 is longitudinally received within outer cannula lumen 126 such that anchor cannula 140 is able to longitudinally translate within and relative to outer cannula 120. In FIGS. 1A-1C and 2A-2E, wall 121 is depicted as being generally tubular, but those skilled in the art will recognize that other suitable wall shapes defining outer cannula 120 could be chosen consistent with the present disclosure.

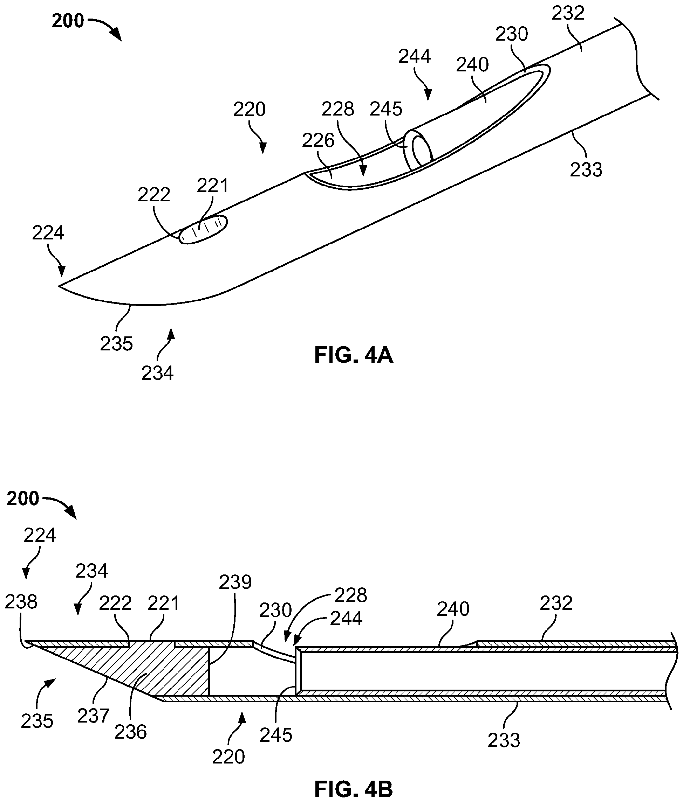

Transverse slot 128 extends radially through wall 121 to connect outer cannula lumen 126 to an external environment surrounding anchor needle 100. Transverse slot 128 may be a port, aperture, notch, or other opening through which tissue can pass into outer cannula lumen 126. In FIGS. 1A-1C and 2A-2E, transverse slot 128 is depicted as having a generally elliptical shape when viewed from above, with circumferential edge 130 that arcs downward from top 132 of outer cannula 120 in side view. However, transverse slot 128 may be any suitable shape or size. Circumferential edge 130 may be planar, for example as depicted in FIGS. 1A-1C and 2A-2E, beveled, for example as depicted in FIGS. 4A and 4B, normal to an exterior surface of outer cannula 120, or variations or combinations thereof.

Outer cannula 120 includes closed needle tip 134. Closed needle tip 134 may generally close off outer cannula lumen 126 distally so as to prevent material from entering outer cannula 120 from a distal direction along the longitudinal axis of outer cannula 120, for example, when the outer cannula is being advanced into tissue, but the user does not want to collect a sample at the initial location of penetration as illustrated in FIGS. 2A-2E. In FIGS. 1A-1C, closed needle tip 134 is formed by folding a plug or cap portion 136 of top 132 towards a portion 137 of bottom 133 to close off outer cannula lumen 126 distally. Plug or cap portion 136 may be formed as a flap cut from top 132 and have a profile shaped to distally block and close off outer cannula lumen 126. To make the embodiment of FIGS. 1A-1C, with plug or cap portion 136 folded towards portion 137, plug or cap portion 136 may then be affixed to an inner surface of wall 121, for example with solder, adhesive, or any other suitable means. Closed needle tip 134 may thus be understood to be substantially permanently closed. Affixing plug or cap portion 136 to an inner surface of wall 121 may help prevent material (e.g. un-targeted tissue) from entering outer cannula 120 from a distal direction along the longitudinal axis of outer cannula 120, as well as prevent material (e.g. sampled tissue) from exiting outer cannula 120 from a proximal direction along the longitudinal axis of outer cannula 120. Outer cannula 120 extends distal to plug or cap portion 136 and terminates in a distal edge 135. In FIGS. 1A-1C and 2A-2E, distal edge 135 is depicted as being beveled to aid entry into desired tissue (e.g. a target lesion), and as slanting downward distally from top 132 to bottom 133. In some embodiments, closed needle tip 134 may take other suitable forms, some of which are discussed in further detail below. In some embodiments, some of which are discussed in further detail below, outer cannula 120 may include an open needle tip rather than closed needle tip 134.

Outer cannula 120 further includes anchor cannula stop 139. Anchor cannula stop 139 extends upward from bottom 133 transverse to the longitudinal axis of outer cannula 120 and into outer cannula lumen 126. Anchor cannula stop 139 is adapted to stop distal longitudinal translation of anchor cannula 140 within outer cannula lumen 126. In FIGS. 1A-1C and 2A-2E, anchor cannula stop 139 is depicted as being formed by folding a portion of bottom 133 toward top 132 at a location proximal to closed needle tip 134 and distal to transverse slot 128. The portion of bottom 133 folded toward top 132 may be a flap cut into bottom 133. In some embodiments, anchor cannula stop 139 may take on other suitable shapes and be made in other suitable manners.

Anchor cannula 140 includes a wall 141 that extends from anchor cannula proximal end 142 to anchor cannula distal end 144. Wall 141 defines an anchor cannula lumen 146. Anchor cannula lumen 146 is adapted to transmit a vacuum to anchor cannula distal end 144. For example, anchor cannula lumen 146 may be open for its length from anchor cannula proximal end 142 to anchor cannula distal end 144, so that a vacuum applied at anchor cannula proximal end 142 can be transmitted to anchor cannula distal end 144. In FIGS. 1A-1C and 2A-2E, wall 141 is depicted as being generally tubular, but those skilled in the art will recognize that other suitable wall shapes defining anchor cannula 140 could be chosen consistent with the present disclosure. Anchor cannula distal end 144 is depicted in FIGS. 1A-1C and 2A-2E as having a distal edge 145 that is a straight planar edge substantially normal to the longitudinal axis of anchor cannula 140. This edge shape may be atraumatic, rather than sharp, which may help to prevent needle stick injuries, for example during tissue sample retrieval. This edge shape may allow anchor cannula 140 to act as a cutting block for outer cannula 120 and circumferential edge 130. However, in some embodiments, anchor cannula distal end 144 may have other edge shapes, including for example beveled, for example as depicted in FIGS. 4A and 4B, and/or slanted, and/or sharpened, for example to assist in or perform tissue sample cutting. In some embodiments where anchor cannula distal end 144 is shaped to assist in or perform tissue sample cutting, outer cannula 120 may act as a cutting block for anchor cannula 140. In some embodiments, anchor cannula distal end 144 may have an edge that is a complex curve.

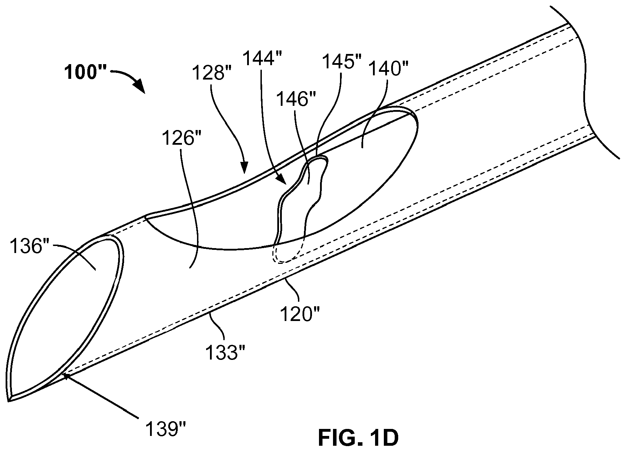

For example, FIG. 1D depicts anchor cannula 100", which includes outer cannula 120" and anchor cannula 140". Outer cannula 120" and anchor cannula 140" can be held and coupled by a cannula support such as cannula support 160.

Outer cannula 120" generally includes the features and functions of outer cannula 120, with certain modifications that will be apparent from the present disclosure to those skilled in the art. Outer cannula 120" includes outer cannula lumen 126", which extends unobstructed to a proximal edge 139" of plug or cap portion 136", such that when outer cannula 120" and anchor cannula 140" translate relative to each other, anchor cannula 140" can contact proximal edge 139". Outer cannula 120" may thus include bottom 133". Bottom 133" lacks a portion folded up into lumen 126" to act as an anchor stop. Rather, proximal edge 139" of plug or cap portion 136" may act as an anchor stop. In this way, outer cannula 120" may act as a cutting block for anchor cannula 140".

Anchor cannula 140" includes anchor cannula distal end 144", which includes distal edge 145". Distal edge 145" is defined by a complex curve that includes a plurality of undulations extending about a circumference of anchor cannula 140". Distal edge 145" is also slanted relative to the longitudinal axis of anchor cannula 140". Distal edge 145" may be slanted at an angle that is complementary to an angle of proximal edge 139". The complex curve of distal edge 145" is depicted in FIG. 1D as further being beveled. This configuration of distal edge 145" may allow anchor cannula 140" to cut or assist in cutting a tissue sample passing through transverse slot 128" of outer cannula 120".

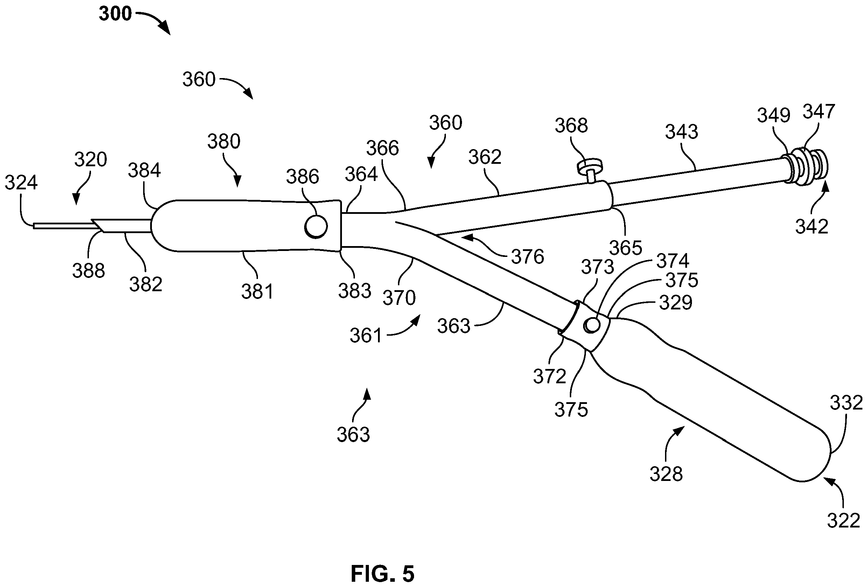

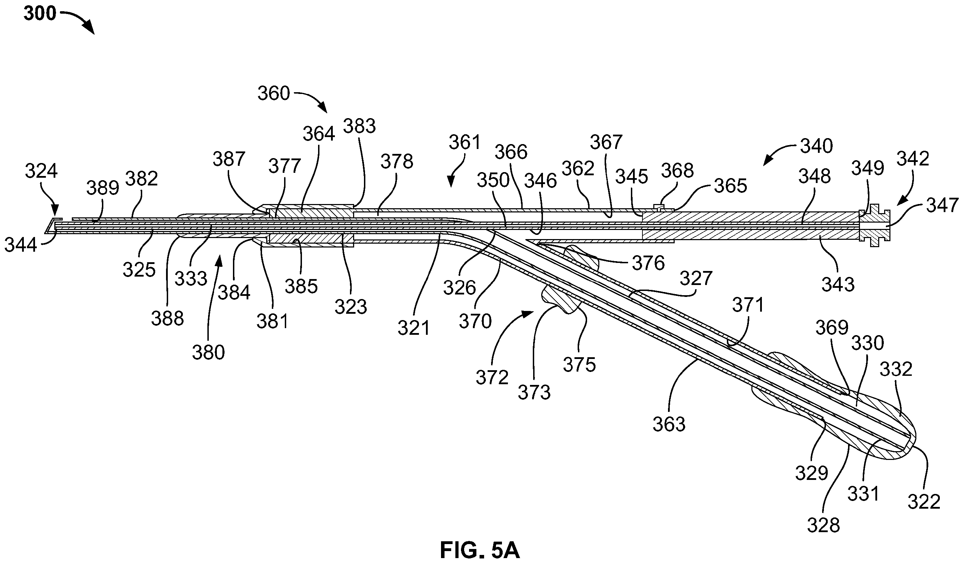

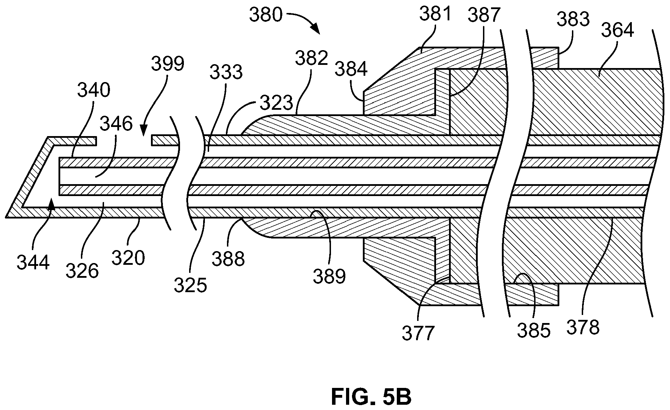

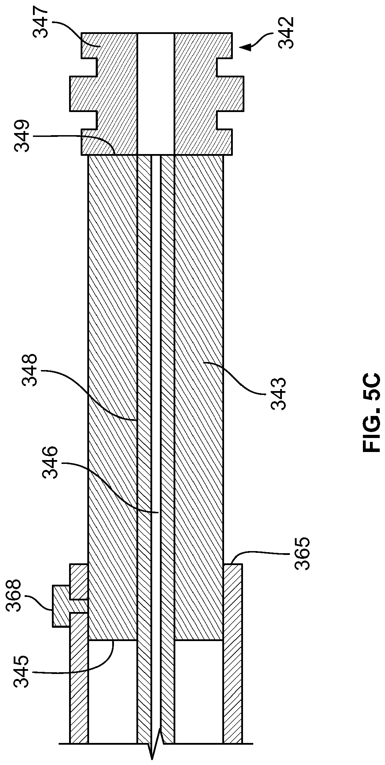

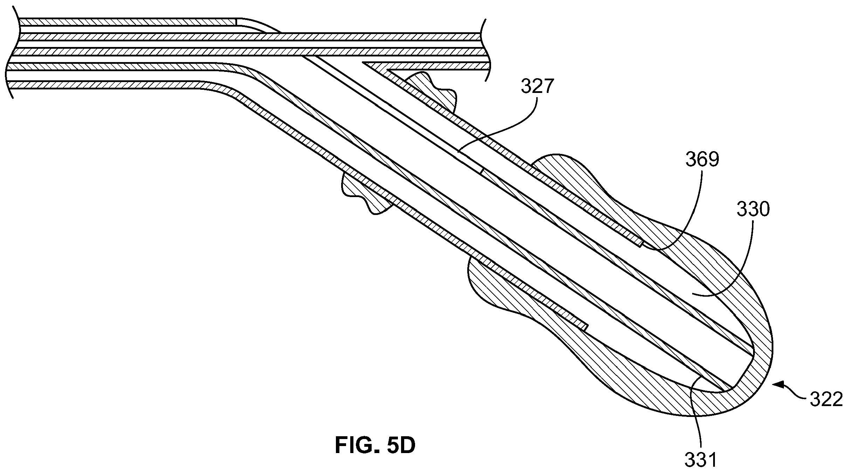

In FIGS. 1A and 1B, cannula support 160 is depicted in schematic form. Cannula support 160 can be embodied in a variety of forms, some of which are described in further detail below with reference to FIGS. 5-6G. Generally, cannula support 160 is adapted to hold and couple together outer cannula 120 and anchor cannula 140 at a position proximal to outer cannula distal end 124 and proximal to anchor cannula distal end 144. For example, in some embodiments, both outer cannula proximal end 122 and anchor cannula proximal end 142 extend proximal to cannula support 160. In other exemplary embodiments, anchor cannula proximal end 142 extends proximal to cannula support 160, but outer cannula proximal end 122 does not. Those skilled in the art may recognize other suitable relationships between anchor cannula proximal end 142 and outer cannula proximal end 122. Anchor cannula 140 can be releasably locked to cannula support 160 so that longitudinal translation of anchor cannula 140 relative to cannula support 160 is alternately allowed and prevented. Outer cannula 120 can also be releasably locked to cannula support 160 so that longitudinal translation of outer cannula 120 relative to cannula support 160 is alternately allowed and prevented. In some configurations, the releasable locking of anchor cannula 140 to cannula support 160 and the releasable locking of outer cannula 120 to cannula support 160 are independent of each other. In other words, in some configurations, anchor cannula 140 can be locked to cannula support 160 while outer cannula 120 is able to longitudinally translate relative to cannula support 160, and outer cannula 120 can be locked to cannula support 160 while anchor cannula 140 is able to longitudinally translate relative to cannula support 160.

A method of operating an anchor needle to remove a tissue sample from a target lesion is described with reference to FIGS. 2A-2E and anchor needle 100. Prior to removing a tissue sample, anchor needle 100 is maneuvered to a desired sampling position relative to target lesion 190. Positioning of anchor needle 100 may for example be conducted using endoscopic ultrasound imaging to monitor a position of the distal section of anchor needle 100 relative to target lesion 190. Generally, to maneuver anchor needle 100 to the desired sample position, a distal section of anchor needle 100 is introduced into target lesion 190, as depicted in FIGS. 2A and 2B. Specifically, outer cannula distal end 124 is initially passed through adjacent tissue 191, with anchor cannula distal end 144 positioned proximally to and following outer cannula distal end 124, for example as depicted in FIG. 2A. Then, outer cannula distal end 124 and anchor cannula distal end 144 continue together to be introduced distally into the patient so as to enter target lesion 190, for example as depicted in FIG. 2B. As the distal section of anchor needle 100 is maneuvered from its position in FIG. 2A to its position in FIG. 2B, outer cannula 120 and anchor cannula 140 are substantially prevented from longitudinally translating relative to each other. In other words, as the distal section of anchor needle 100 is maneuvered to the desired sampling position, longitudinal translation of outer cannula 120 causes corresponding longitudinal translation of anchor cannula 140, so that the position of outer cannula distal end 124 relative to anchor cannula distal end 144 remains substantially fixed. Some examples of configurations suitable to substantially prevent such relative longitudinal translation are described below in connection with FIGS. 5-6G. Anchor needle 100 may be determined to be positioned at a desired sampling position relative to target lesion 190 when outer cannula distal end 124 and/or anchor cannula distal end 144 are positioned near a portion of target lesion 190 that is intended to be sampled.

Additionally, as anchor needle 100 is maneuvered to the desired sampling position relative to target lesion 190, outer cannula 120 and anchor cannula 140 are in a blocking configuration, so that no material from the adjacent tissue 191 is collected into the lumen during initial positioning as shown and described. In an exemplary blocking configuration depicted in FIGS. 2A and 2B, anchor cannula 140 is longitudinally positioned relative to outer cannula 120 so as to completely internally cover transverse slot 128. In other words, as anchor needle 100 is maneuvered to the desired sampling position relative to target lesion 190, anchor cannula 140 blocks transverse slot 128 to prevent material (e.g. adjacent tissue 191 and portions of target lesion 190) from passing through transverse slot 128 and into outer cannula lumen 126. For example, in FIGS. 2A and 2B, anchor cannula distal edge 145 is positioned so as to abut anchor cannula stop 139, with cannula distal edge 145 thus being positioned distal to a distal-most portion of circumferential edge 130.

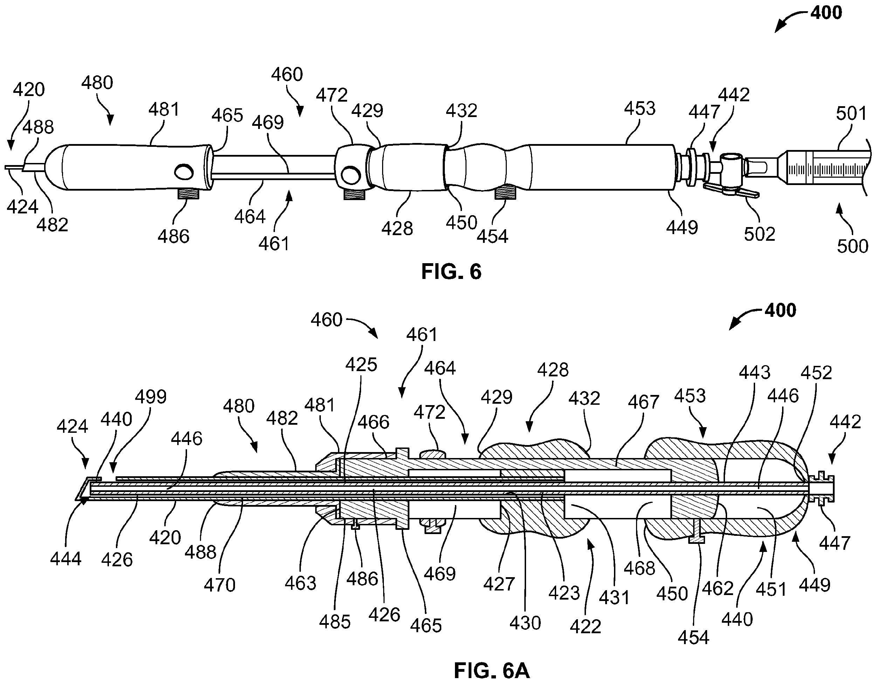

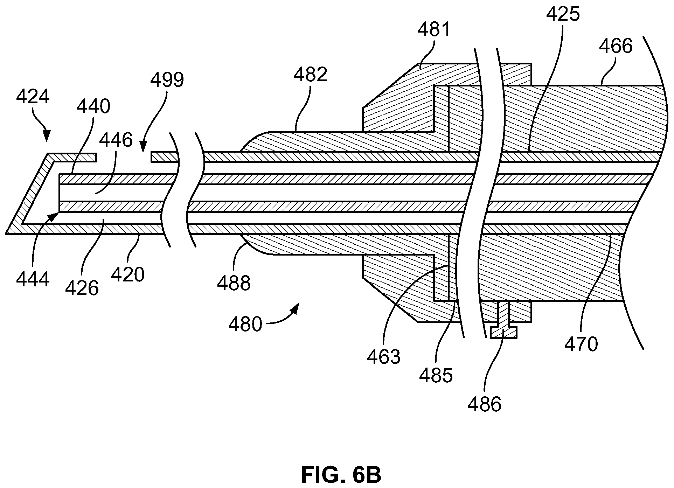

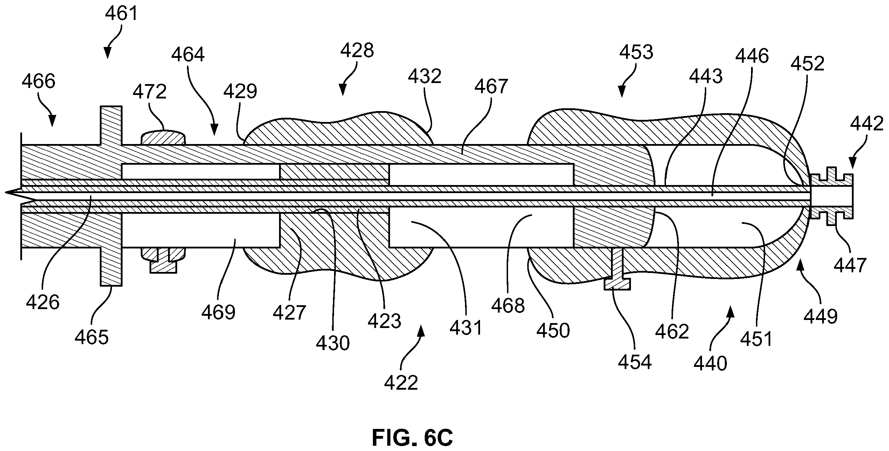

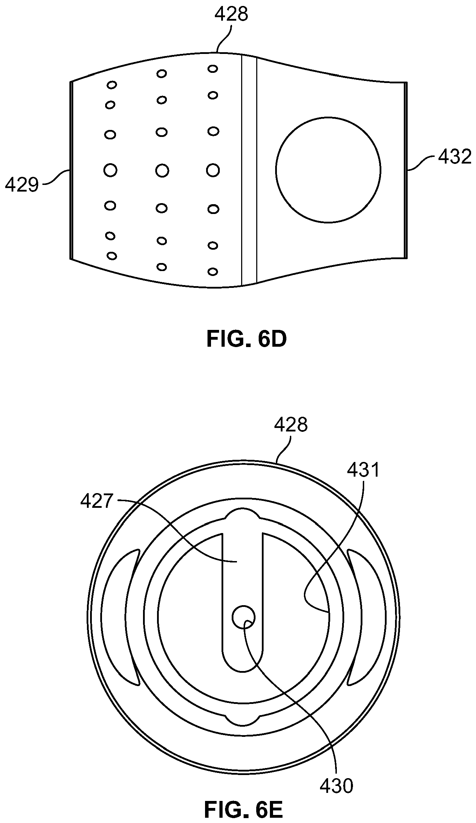

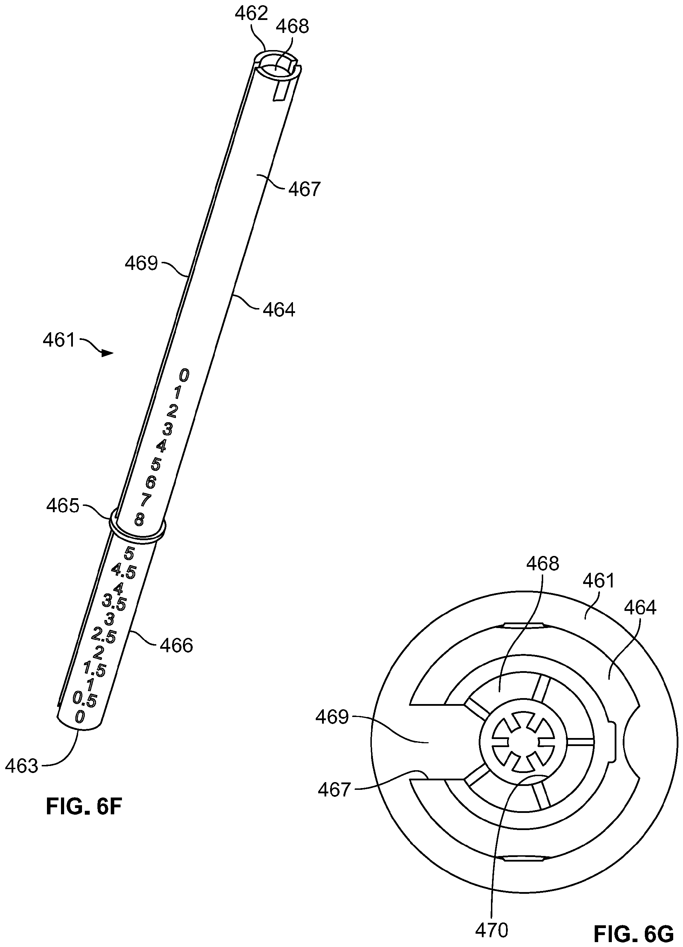

After anchor needle 100 reaches its desired sampling position, e.g. its position in FIG. 2B, anchor cannula 140 is releasably locked to cannula support 160 so that longitudinal translation of anchor cannula 140 relative to cannula support 160 is prevented. Vacuum 196 is applied to anchor cannula lumen 146. Vacuum 196 may be applied by turning on a vacuum source connected to anchor cannula proximal end 142. A suitable vacuum source could include a negative pressure generator and a valve positioned between the negative pressure generator and anchor cannula proximal end 142 so that a vacuum can be applied to anchor cannula proximal end 142 when the valve is opened and not applied to anchor cannula proximal end 142 when the valve is closed. An example of such a vacuum source is described below with reference to FIG. 6. However other suitable vacuum sources could be used to apply vacuum 196. Outer cannula 120 is not at this time locked to cannula support 160, and thus outer cannula 120 is able to longitudinally translate relative to anchor cannula 140 and cannula support 160.

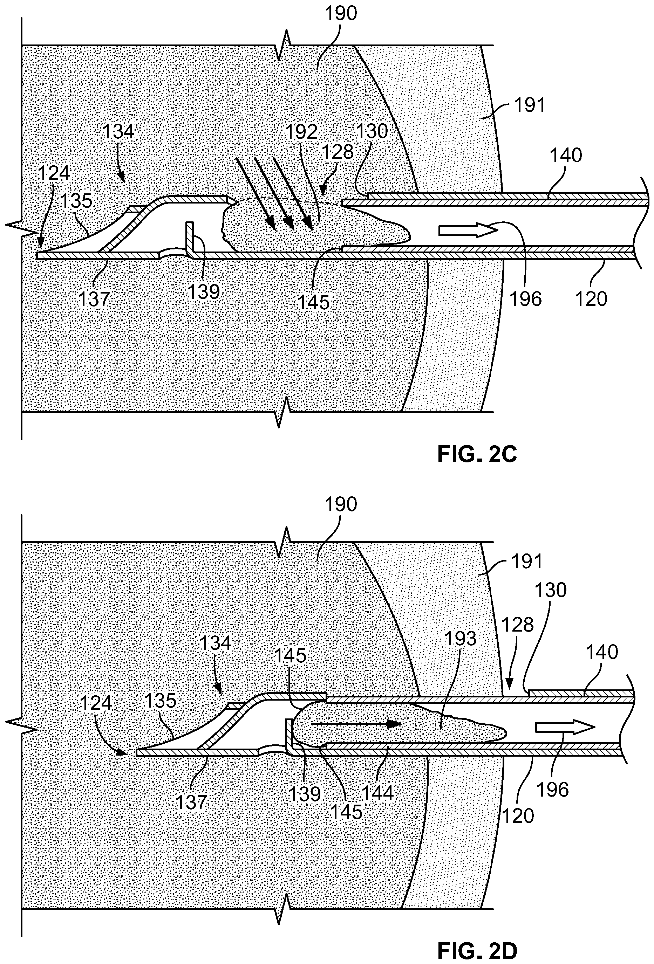

Next, outer cannula 120 and anchor cannula 140 are placed in an exposing configuration. To place outer cannula 120 and anchor cannula 140 in the exemplary exposing configuration depicted in FIG. 2C, outer cannula 120 is longitudinally translated distally relative to anchor cannula 140 and cannula support 160, so that anchor cannula 140 no longer internally covers transverse slot 128. Tissue 192 is thus able to pass through transverse slot 128 and into outer cannula lumen 126. With outer cannula 120 and anchor cannula 140 in the exposing configuration, and anchor cannula 140 locked to cannula support 160, the anchor needle is arranged in a tissue-anchoring configuration. With the anchor needle arranged in the tissue-anchoring configuration, vacuum 196 draws tissue 192 through transverse slot 128, into outer cannula lumen 126, and toward anchor cannula distal end 144, thereby anchoring tissue 192 to anchor cannula 140. Anchoring tissue 192 to anchor cannula 140 may include drawing tissue 192 partially into anchor cannula lumen 146 as depicted in FIG. 2C. This vacuum-aided tissue-anchoring effect holds tissue 192 in place during sample cutting, preventing tissue 192 from being pushed away from anchor needle 100 as may occur with conventional biopsy needles as discussed above.

Next, outer cannula 120 is longitudinally translated proximally relative to anchor cannula 140 and cannula support 160, so that transverse slot 128 again becomes internally covered by anchor cannula 140, for example as depicted in FIG. 2D. In the process of transverse slot 128 becoming internally covered by anchor cannula 140, tissue sample 193 is cut and severed from tissue 192. The cutting of tissue sample 193 may occur due to a pinching, squeezing, and/or shearing force that is applied to a portion of tissue 192 that is within transverse slot 128, by and between portions of transverse slot circumferential edge 130 and anchor cannula distal edge 145. The pinching, squeezing, and/or shearing force may be generated when outer cannula 120 (and thus transverse slot 128) are longitudinally translated proximally relative to anchor cannula 140. One or both of circumferential edge 130 or distal edge 145 may be configured to facilitate the cutting that is caused by the pinching, squeezing, and/or shearing force. For example, one or both of circumferential edge 130 or distal edge 145 may be an angled planar edge, a beveled edge, a sharpened edge, and/or having cutting characteristics recognized by those of skill in the art. For example, as depicted in FIGS. 1A-1C and 2A-2E, circumferential edge 130 is a planar edge angled inwards toward transverse slot 128, while distal edge 145 is a straight planar edge substantially normal to the longitudinal axis of anchor cannula 140. Anchor cannula distal edge 145 may thus act as a cutting block for outer cannula 120 and circumferential edge 130. Vacuum 196 may continue to be applied while outer cannula 120 is longitudinally translated proximally relative to anchor cannula 140 and cannula support 160.

Tissue sample 193, once severed, becomes positioned within anchor cannula lumen 146, for example as depicted in FIG. 2D. Vacuum 196 continues to be applied to anchor cannula lumen 146, which aids in drawing tissue sample 193 into anchor cannula lumen 146. If additional tissue samples are desired, outer cannula 120 can again be longitudinally translated distally so that another portion of tissue can be drawn through transverse slot 128. Outer cannula 120 can then again be longitudinally translated proximally to cut and sever another tissue sample. Outer cannula 120 can be repeatedly longitudinally reciprocated in a similar manner until sufficient tissue has been collected within anchor cannula lumen 146, including that the relative depth and/or angle of the device within the target lesion 190 can be varied during reciprocation. Vacuum 196 may be continuously applied to anchor cannula distal end 144 as outer cannula 120 is repeatedly longitudinally reciprocated.

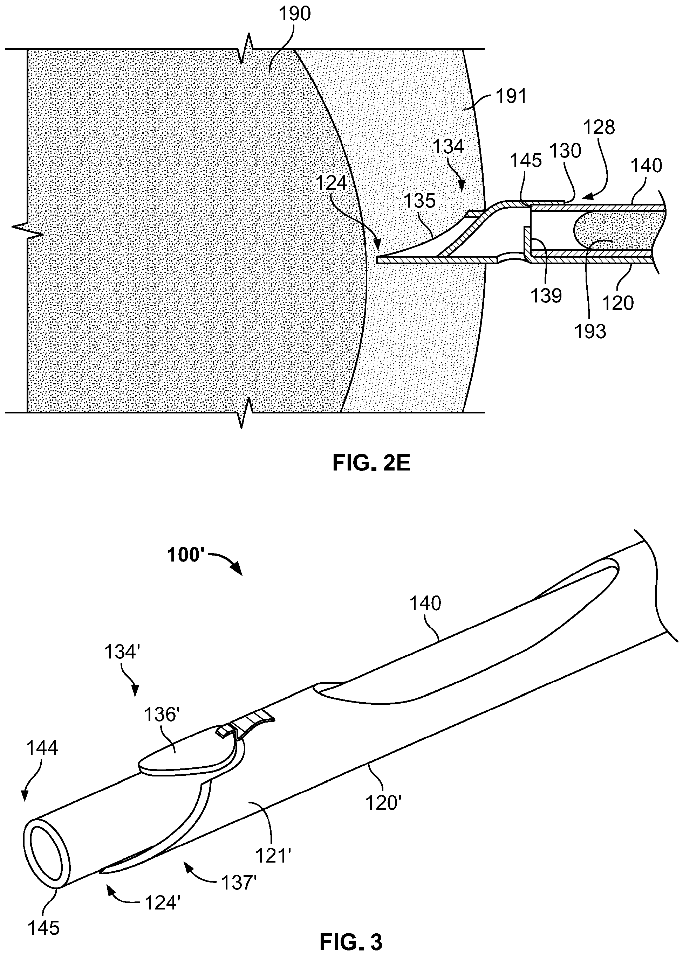

When a desired amount of tissue has been collected within anchor cannula lumen 146, tissue sample 193 (or multiple tissue samples) is/are retrieved from anchor cannula 140. In some embodiments, including when closed needle tip 134 is substantially permanently closed, for example as described in connection with FIGS. 1A-1C, tissue samples are retrieved by proximally withdrawing anchor cannula 140 from outer cannula 120 so that anchor cannula distal end 144 can be accessed. To do so, outer cannula 120 is longitudinally positioned so that anchor cannula 140 internally covers transverse slot 128, for example as depicted in FIGS. 2D and 2E. Application of vacuum 196 to anchor lumen 146 is stopped, and anchor cannula 140 is unlocked from cannula support 160 so that anchor cannula 140 can longitudinally translate relative to cannula support 160. Outer cannula 120 and anchor cannula 140 are together withdrawn from target lesion 190, for example as shown in FIG. 2E. As outer cannula 120 and anchor cannula 140 are together withdrawn from target lesion 190, they are again substantially prevented from longitudinally translating relative to each other, for example in a manner similar to that described above in connection with FIGS. 2A and 2B. In other words, longitudinal translation of outer cannula 120 causes corresponding longitudinal translation of anchor cannula 140, so that the position of outer cannula distal end 124 relative to anchor cannula distal end 144 remains substantially fixed. Then, outer cannula 120 is releasably locked to cannula support 160 so that longitudinal translation of outer cannula 120 relative to cannula support 160 is prevented. Anchor cannula 140 is then withdrawn so that it longitudinally translates proximally relative to outer cannula 120 and cannula support 160, until anchor cannula distal end 144 is withdrawn from cannula support 160 and anchor cannula 140 is separated from cannula support 160 and outer cannula 120. Tissue sample 193 may then be retrieved from anchor cannula lumen 146 in any suitable manner, for example by applying a positive pressure to anchor cannula proximal end 142 to eject tissue sample 193 out from anchor cannula distal end 144 and into a sample receiving container.

Some embodiments include a closed needle tip that is not substantially permanently closed. For anchor needles having a closed needle tip that is not substantially permanently closed, an alternative method can be used to retrieve tissue sample 193 (or multiple tissue samples) from anchor cannula 140.

An exemplary anchor needle 100' with a closed needle tip 134' that is not substantially permanently disclosed is illustrated in FIG. 3. Anchor needle 100' includes outer cannula 120' and anchor cannula 140. Outer cannula 120' generally includes the features and functions of outer cannula 120, with certain modifications that will be apparent from the present disclosure to those skilled in the art. Closed needle tip 134' may be configured to transition between a closed configuration and an open configuration. For example, plug or cap portion 136' may not be affixed to an inner surface of wall 121' when folded towards portion 137'. Instead, at least plug or cap portion 136' of outer cannula 120' could be formed as a movable cap from a shape memory material (e.g. a nickel titanium alloy) that is configured or biased to assume or return to a folded-down shape resulting in a closed configuration, for example such as is shown in FIGS. 1A-1C. The closed configuration may help prevent material (e.g. un-targeted tissue) from entering outer cannula 120' from a distal direction along the longitudinal axis, and may prevent material (e.g. sampled tissue) from exiting outer cannula 120' from a proximal direction along the longitudinal axis of outer cannula 120'. In such embodiments, sufficient pressure applied on a proximal-face of plug or cap portion 136' may cause plug or cap portion 136' to move from the folded-down shape and the closed configuration, for example as shown in FIGS. 1A-1C, to an extended shape and an open configuration, for example as shown in FIG. 3. The pressure may be applied by anchor cannula distal edge 145 to allow anchor cannula distal end 142 to push plug or cap portion 136' out of the way, such that a portion of anchor cannula 140 is able to extend distal to outer cannula distal end 124'. Then, when anchor cannula distal edge 145 is withdrawn proximal to plug or cap portion 136', the shape memory nature of plug or cap portion 136' will cause it to return to the folded-down shape and the closed configuration. Outer cannula 120' may omit anchor cannula stop 139 so that anchor cannula 100' is able to be operated in the disclosed manner (e.g. so that anchor cannula distal end 142 is able to longitudinally translate distally far enough relative to outer cannula 120' to push plug or cap portion 136' out of the way).

Embodiments that have a closed needle tip 134' that is not substantially permanently closed, and that can instead transition between a closed configuration and an open configuration, may provide for convenient sample removal. For example the user would not need to withdraw anchor cannula 140 proximally out of outer cannula 120' to access a sample in anchor cannula lumen 146. Instead, the user could simply withdraw outer cannula 120' and anchor cannula 140 together from the patient, place anchor needle 100' in the open configuration, for example as shown in FIG. 3, and remove the sample from anchor cannula distal end 144 while anchor cannula distal edge 145 is extending distal to plug or cap portion 136'. Because the user would not need to withdraw anchor cannula 140 from outer cannula 120' to retrieve the sample, the risk of dropping or bending anchor cannula 140 may be reduced.

Although methods of operating anchor needles have been generally described with reference to anchor needle 100, other anchor needle designs can also be used within the method described with reference to FIGS. 2A-2E, with certain modifications that will be apparent from the present disclosure to those skilled in the art from the present disclosure. For example, anchor needle 200 includes outer cannula 220 and anchor cannula 240, which can be held and coupled by a cannula support such as cannula support 160.

Outer cannula 220 generally includes the features and functions of outer cannula 120, with certain modifications that will be apparent from the present disclosure to those skilled in the art.

Outer cannula 220 includes closed needle tip 234. Closed needle tip 234 is closed by inserting plug 236 into outer cannula lumen 226 at outer cannula distal end 224, for example as depicted in FIGS. 4A and 4B. Plug 236 could be formed of solder or other suitable material. Plug 236 is shaped to be received in and substantially conform to a distal section of outer cannula lumen 226, and extends from plug distal edge 237 to plug proximal edge 239. Plug distal edge 237 sits flush with wall distal edge 238 to together form outer cannula distal edge 235. In FIGS. 4A and 4B, outer cannula distal edge 235 slants downward proximally from top 232 to bottom 233. However in some embodiments, outer cannula distal edge 235 could instead slant downward distally from top 232 to bottom 233, for example similar to distal edge 135 in FIGS. 1A-1C and 2A-2E. Plug proximal edge 239 is positioned between wall distal edge 238 and transverse slot 228, and extends upward through outer cannula lumen 226. Plug proximal edge 239 acts substantially as an anchor cannula stop, for example similar to anchor cannula stop 139. Plug 236 includes radial projection 221, which is shaped and positioned to be received in retaining slot 222 of outer cannula 220 when plug 236 is received in outer cannula lumen 226. Retaining slot 222 extends transversely through a wall defining outer cannula 220. Engagement between radial projection 221 and retaining slot 222, for example as depicted in FIGS. 4A and 4B, holds plug 236 in place in outer cannula 220. In some embodiments, radial projection 221 and retaining slot 222 may be omitted, and plug 236 could be secured in a variety of known manners, including for example by compression inside outer cannula 220.

Outer cannula 220 also includes circumferential edge 230. Circumferential edge 230 is beveled, as depicted in FIGS. 4A and 4B. The beveled nature of circumferential edge 230 may enhance the cutting action of anchor needle 200 by producing a shearing effect on tissue located between circumferential edge 230 and anchor cannula 240 when outer cannula 220 is longitudinally translated proximally relative to anchor cannula 240.

Anchor cannula 240 generally includes the features and functions of anchor cannula 140, with certain modifications that will be apparent from the present disclosure to those skilled in the art. Anchor cannula 240 includes anchor cannula distal edge 245. Anchor cannula distal edge 245 is inwardly beveled, as depicted in FIGS. 4A and 4B. The beveled nature of anchor cannula distal edge 245 can enhance the cutting action of anchor needle by producing a shearing effect on tissue located between anchor cannula distal edge 245 and circumferential edge 230 when outer cannula 220 is longitudinally translated proximally relative to anchor cannula 240.

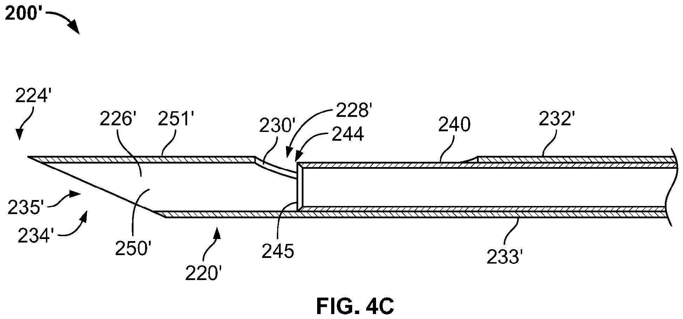

In some embodiments, an anchor needle may include an outer cannula with an open needle tip rather than a closed needle tip. For example, FIG. 4C illustrates anchor needle 200'. Anchor needle 200' generally include the features and functions of anchor needle 200, but includes outer cannula 220' rather than outer cannula 220. Outer cannula 220' and anchor cannula 240 can be held and coupled by a cannula support such as cannula support 160.

Outer cannula 220' includes open needle tip 234' distal to transverse slot 228'. Open needle tip 234' is similar to closed needle tip 234, but does not include plug 236 or retaining slot 222. Instead, open needle tip 234' includes distal aperture 250' and continuous upper distal wall section 251'. Distal aperture 250' connects outer cannula lumen 226' to an external environment of outer cannula 220', such that material can enter outer cannula 220' from a distal direction by passing through distal aperture 250' and into outer cannula lumen 226'. In FIG. 4C, outer cannula distal edge 235' slants downward proximally from top 232' to bottom 233'. However in some embodiments, outer cannula distal edge 235' could instead slant downward distally from top 232' to bottom 233', for example similar to distal edge 135 in FIGS. 1A-1C and 2A-2E.

Outer cannula 220' also includes circumferential edge 230'. Circumferential edge 230' is beveled, as depicted in FIG. 4C. The beveled nature of circumferential edge 230' may enhance the cutting action of anchor needle by producing a shearing effect on tissue located between circumferential edge 230' and anchor cannula 240 when outer cannula 220' is longitudinally translated proximally relative to anchor cannula 240.

Other embodiments of anchor needles that include an outer cannula with an open needle tip may also be suitable.

Anchor needles that include an outer cannula with an open needle tip may generally be operated in a manner analogous to anchor needles that include an outer cannula with a closed tip. Anchor needles that include an outer cannula with an open needle tip may be particularly of use in procedures during which the clinician is able to readily differentiate unwanted tissue from the targeted tissue.

The present disclosure has so far focused mainly on features and functions of distal sections of anchor needles, for example in the context of distal sections of anchor needle 100 and anchor needle 200. Features and functions of proximal sections of anchor needles, for example cannula supports, cannula handles, etc., that may allow for implementation of the present disclosure's methods of using anchor needles, will be further described with reference to FIGS. 5-6G.

FIGS. 5, 5A, 5B, 5C, and 5D illustrate anchor needle 300. Anchor needle 300 is adapted to implement the present disclosure's methods of using an anchor needle described above with reference to FIGS. 2A-2E. Anchor needle 300 includes outer cannula 320, anchor cannula 340, cannula support 360, and sheath assembly 380. As will become apparent to those skilled in the art, anchor needle 300 provides parallel controls for outer cannula 320 and anchor cannula 340, which may result in the benefit that the total length of anchor needle 300 can be made substantially the same as conventional biopsy needles used in endoscopic ultra-sound fine needle aspiration.

Outer cannula 320 may be generally embodied as, and have the features and functions of, outer cannula 120, outer cannula 120', or outer cannula 220, with certain additions and/or modifications proximal to transverse slot 128 or transverse slot 228 that will be apparent from the present disclosure to those skilled in the art.

Outer cannula 320 includes a wall 321 that extends from outer cannula proximal end 322 to outer cannula distal end 324. Wall 321 defines an outer cannula lumen 326. Outer cannula 320 further includes outer cannula proximal section 323 and outer cannula distal section 325. Outer cannula proximal section 323 and outer cannula distal section 325 are connected together at connection 333 such that longitudinal translation of one causes longitudinal translation of the other. Outer cannula lumen 326 extends continuously through outer cannula proximal section 323 and outer cannula distal section 325. Outer cannula proximal section 323 is more flexible than outer cannula distal section 325. For example, outer cannula proximal section 323 may be formed from a suitable plastic, and outer cannula distal section 325 may be formed from a suitable stainless steel. Outer cannula proximal section 323 may have a larger outer diameter than outer cannula distal section 325.

Outer cannula proximal section 323 includes receiving slot 327 that extends longitudinally along a portion of outer cannula proximal section 323. Receiving slot 327 extends radially through wall 321 to connect an exterior of outer cannula 320 to outer cannula lumen 326. Receiving slot 327 is sized, shaped, and positioned so that anchor cannula 340 can pass through receiving slot 327 and into outer cannula lumen 326. Other forms of openings could be used so that anchor cannula 340 can pass through them and into outer cannula lumen 326. For example, rather than receiving slot 327, outer cannula 320 could include a planar orifice (not shown) located where the distal edge of receiving slot 327 is shown in FIGS. 5A and 5D. The planar orifice could be configured so that when outer cannula 320 is received in cannula support 360 as generally depicted in FIGSS. 5,5A, and 5D, the planar orifice faces substantially towards anchor cannula holder proximal end 365. The planar orifice may include a rubber seal to grip the exterior of anchor cannula 340.

Outer cannula 320 also includes cutting handle 328. Cutting handle 328 extends from outer cannula proximal end 322 to cutting handle distal end 329 and defines cutting handle lumen 330. A proximal portion of outer cannula proximal section 323 is positioned within and extends through cutting handle lumen 330 such that outer cannula proximal section proximal edge 331 attaches to cutting handle proximal end 332. Longitudinal translation of cutting handle 328 causes corresponding longitudinal translation of outer cannula proximal section 323 and outer cannula distal section 325.

Anchor cannula 340 may be generally embodied as, and have the features and functions of, anchor cannula 140 or anchor cannula 240, with certain additions and/or modifications towards anchor cannula proximal end 142 or anchor cannula proximal end 342 that will be apparent from the present disclosure to those skilled in the art.

Anchor cannula 340 extends from anchor cannula proximal end 342 to anchor cannula distal end 344 and defines anchor cannula lumen 346 between anchor cannula proximal end 342 and anchor cannula distal end 344. Anchor cannula 340 includes anchor handle 343, which coaxially surrounds anchor cannula proximal section 348 from anchor handle distal end 345 to anchor handle proximal end 349. Anchor cannula 340 and anchor handle 343 are connected to each other such that longitudinal translation of one causes longitudinal translation of the other. Anchor cannula handle 343 has a larger outer diameter than anchor cannula proximal section 348. Anchor cannula 340 further includes a vacuum connector 347 connected to anchor cannula proximal section 348 and anchor handle proximal end 349. Vacuum connector 347 connects to anchor cannula lumen 346 so that a vacuum can be applied through vacuum connector 347 to anchor cannula lumen 346 and transmitted to anchor cannula distal end 344. Vacuum connector 347 may for example be a Luer connector. Anchor cannula 340 further includes anchor cannula distal section 350, which has an outer diameter and shape adapted to pass through receiving slot 327 into outer cannula lumen 326. Anchor cannula proximal section 348 and anchor cannula distal section 350 may in some embodiments be formed as a single piece of suitable stainless steel.

Cannula support 360 may be an implementation of, or a form for, cannula support 160.

Cannula support 360 includes Y-shaped structure 361. Y-shaped structure 361 is formed from anchor cannula holder 362, outer cannula holder 363, and cannula coupler 364, each of which form a respective branch of Y-shaped structure 361. Y-shaped structure 361 may in some embodiments be formed as a single unitary piece.

Anchor cannula holder 362 extends from anchor cannula holder proximal end 365 to anchor cannula holder distal end 366 and defines anchor cannula holder lumen 367 between anchor cannula holder proximal end 365 and anchor cannula holder distal end 366. Anchor cannula holder lumen 367 has an inner diameter at anchor cannula holder proximal end 365 that is configured to receive and conform to the outer diameter of anchor handle 343 such that anchor handle 343 can longitudinally translate within anchor cannula holder lumen 367 relative to anchor cannula holder 362.

Cannula support 360 further includes anchor cannula lock 368 connected to anchor cannula holder 362. Anchor cannula lock 368 is adapted to releasably lock longitudinal translation of anchor cannula 340 relative to cannula support 360. In FIGS. 5, 5A, and 5C, anchor cannula lock 368 is depicted as a thumbscrew passing through anchor cannula holder 362 and into anchor cannula holder lumen 367 so as to engage with anchor handle 343. The thumbscrew may be turned in a first direction to apply pressure to the exterior surface of anchor handle 343 and thereby lock longitudinal translation of anchor cannula 340 relative to cannula support 360. The thumbscrew may be turned in an opposite second direction to remove pressure from the exterior surface of anchor handle 343 and thereby unlock anchor cannula 340 relative to cannula support 360 so that anchor cannula 340 is allowed to longitudinally translate relative to cannula support 360. However other suitable mechanisms for releasably locking longitudinal translation of anchor cannula 340 relative to cannula support 360 may be apparent to those skilled in the art. For example, the thumbscrew may be replaced by a push-button locking mechanism or fold-over latch mechanism, each of which works via interlocking teeth or cogs, or by an over-center design which locks due to friction.

Outer cannula holder 363 extends from outer cannula holder proximal end 369 to outer cannula holder distal end 370 and defines outer cannula holder lumen 371 between outer cannula holder proximal end 369 and outer cannula holder distal end 370. Outer cannula holder lumen 371 has an inner diameter that is configured to receive outer cannula proximal section 323 such that outer cannula 320 can longitudinally translate within outer cannula holder lumen 371 relative to outer cannula holder 363. Outer cannula holder 363 has an exterior diameter that is sized and shaped to be received within and conform to cutting handle lumen 330, such that cutting handle 328 can longitudinally translate along and relative to outer cannula holder 363. For example, when cutting handle 328 is longitudinally translated in a distal direction relative to outer cannula holder 363, progressively more of outer cannula holder 363 is received within cutting handle lumen 330. Similarly, when cutting handle 328 is longitudinally translated in a proximal direction relative to outer cannula holder 363, progressively less of outer cannula holder 363 is received within cutting handle lumen 330. Longitudinal translation of cutting handle 328 relative to outer cannula holder 363 causes corresponding longitudinal translation of outer cannula proximal section 323 within outer cannula holder lumen 371.

Cannula support 360 further includes cutting handle lock 372 connected to outer cannula holder 363. Cutting handle lock 372 is adapted to longitudinally translate along outer cannula holder 363 and releasably lock to outer cannula holder 363 at a plurality of longitudinal positions to set a maximum distal longitudinal translation of outer cannula 320 relative to the cannula support 360. In FIGS. 5, 5A, and 5D, cutting handle lock 372 includes locking ring 373 and thumbscrew 374. Locking ring 373 is adapted to at least partially coaxially surround and receive outer cannula holder 363 for longitudinal translation along and relative to outer cannula holder 363. Thumbscrew 374 passes through locking ring 373 so as to engage with outer cannula holder 363. Thumbscrew 374 may be turned in a first direction to apply pressure to the exterior surface of outer cannula holder 363 and thereby lock longitudinal translation locking ring 373 to cannula support 360. The thumbscrew may be turned in an opposite second direction to remove pressure from the exterior surface of outer cannula holder 363 and thereby unlock locking ring 373 relative to cannula support 360 so locking ring 373 is allowed to longitudinally translate relative to cannula support 360. However other suitable mechanisms for releasably locking longitudinal translation of locking ring 373 relative to cannula support 360 may be apparent to those skilled in the art. For example, the thumbscrew may be replaced by a push-button locking mechanism or fold-over latch mechanism, each of which works via interlocking teeth or cogs, or by an over-center design which locks due to friction.

Locking ring 373 is shaped and sized to engage with cutting handle distal end 329 so that cutting handle 328 is prevented from longitudinally translating distally past locking ring proximal edge 375 when locking ring 373 is locked to outer cannula holder 363. Cutting handle lock 372 can thus be used to set a maximum distal longitudinal translation of outer cannula 320 relative to cannula support 360. For example, when locking ring 373 is locked at its proximal-most position, e.g. as depicted in FIG. 5, outer cannula 320 is prevented from longitudinally translating distal relative to cannula support 360 at all. When locking ring 373 is locked at a more distal position, e.g. as depicted in FIGS. 5A and 5D, outer cannula 320 is allowed to longitudinally translate distal relative to cannula support 360 for a distance substantially equivalent to the distance between locking ring proximal edge 375 and cutting handle distal end 329, i.e. until cutting handle distal end 329 abuts locking ring proximal edge 375. Cutting handle 328 (and thus outer cannula 320) may thus be longitudinally translated distally and proximally relative to cannula support 360 in a reciprocating manner.

Anchor cannula holder 362 and outer cannula holder 363 join together at cannula coupler proximal end 376 of cannula coupler 364. Cannula coupler 364 extends from cannula coupler proximal end 376 to cannula coupler distal end 377, and defines cannula coupler lumen 378 between cannula coupler proximal end 376 and cannula coupler distal end 377. Cannula coupler lumen 378 connects at its proximal end to the respective distal ends of anchor cannula holder lumen 367 and outer cannula holder lumen 371, such that outer cannula 320 and anchor cannula 340 can be routed into anchor cannula holder lumen 367 and coupled together therein, as depicted for example in FIGS. 5, 5A and 5D.

Anchor needle 300 can be operated to introduce outer cannula distal end 324 and anchor cannula distal end 344 together to a target lesion, or withdraw them together from a target lesion, while keeping the transverse slot 399 of outer cannula 320 internally covered by anchor cannula 340, for example in order to implement aspects of the method described with reference to FIGS. 2A-2E. When outer cannula 320 and anchor cannula 340 are coupled together within cannula coupler lumen 378, such that anchor cannula distal section 350 passes through receiving slot 327 and into outer cannula holder lumen 371, friction exists between anchor cannula distal section 350 and outer cannula proximal section 323. That friction is sufficient that when anchor cannula lock 368 is unlocked, and outer cannula 320 is longitudinally translated relative to cannula support 360 (e.g. due to longitudinal translation of cutting handle 328), outer cannula 320 and anchor cannula 340 are substantially prevented from longitudinally translating relative to each other. Thus when anchor cannula lock 368 is unlocked, longitudinal translation of outer cannula 320 causes corresponding longitudinal translation of anchor cannula 340, for example in order to introduce outer cannula distal end 324 and anchor cannula distal end 344 together into a target lesion or withdraw them together from a target lesion, with the transverse slot 399 of outer cannula 320 blocked by anchor cannula 340.

Additionally, anchor needle 300 can be operated to reciprocate outer cannula 320 so as to alternately lock anchor cannula 340 to target tissue and cut a sample from the locked target tissue, for example in order to implement aspects of the method described with reference to FIGS. 2A-2E. Specifically, the friction between anchor cannula distal section 350 and outer cannula proximal section 323 is low enough that when anchor cannula lock 368 is locked to anchor cannula holder 362, outer cannula 320 is able to longitudinally translate distally and proximally relative to both anchor cannula 340 and cannula support 360. This may for example allow outer cannula 320 to be reciprocated to alternately (1) uncover the transverse slot 399 of outer cannula 320 so anchor cannula distal end 344 can anchor tissue, and (2) cover the transverse slot 399 of outer cannula 320 in order to cut a tissue sample.

Moreover, anchor needle 300 can be operated to retrieve a tissue sample in anchor cannula lumen 346, for example in order to implement aspects of the method described with reference to FIGS. 2A-2E. Specifically, the friction between anchor cannula distal section 350 and outer cannula proximal section 323 is also low enough that, when anchor cannula lock 368 is unlocked, anchor cannula 340 can be completely withdrawn proximally from outer cannula 320 and cannula support 360, for example if the user holds cutting handle 328 in position relative to cannula support 360 during the withdrawal. This may for example allow the user to retrieve a tissue sample in anchor cannula lumen 346 from anchor cannula distal end 344.

In embodiments (e.g. as described above) in which outer cannula 320 includes a planar orifice rather than receiving slot 327, the planar orifice and/or its rubber seal may be sized and shaped to provide a similar friction effect with outer cannula 320 as is provided by receiving slot 327.

Those skilled in the art will recognize how the features and functions of outer cannula 320, anchor cannula 340, and cannula support 360 can be readily used to implement the method of operating an anchor needle to remove a tissue sample from a target lesion described with reference to FIGS. 2A-2E.

Anchor needle 300 further includes sheath assembly 380. Sheath assembly 380 includes sheath extender 381 and sheath 382. Sheath extender 381 extends from sheath extender proximal end 383 to sheath extender distal end 384. Sheath extender lumen 385 is defined by sheath extender 381 and extends from sheath extender proximal end 383 to a point proximal to sheath extender distal end 384. Sheath extender lumen 385 is sized and shaped to receive cannula coupler 364, such that cannula coupler 364 can longitudinally translate within and relative to sheath extender lumen 385. Sheath extender 381 may include a sheath extender lock 386 to releasably lock sheath extender 381 to cannula coupler 364, so as to alternately prevent and allow longitudinal translation of sheath extender 381 relative to cannula coupler 364. Sheath extender lock 386 may be a mechanism similar to anchor cannula lock 368.

Sheath 382 extends from sheath proximal end 387 to sheath distal end 388 and defines sheath lumen 389 between sheath proximal end 387 and sheath distal end 388. Sheath lumen 389 is adapted to receive outer cannula 320 such that outer cannula 320 can longitudinally translate within and relative to sheath lumen 389. Sheath proximal end 387 is connected to sheath extender 381 such that longitudinal translation of sheath extender 381 causes corresponding longitudinal translation of sheath 382. Sheath extender 381 may coaxially surround sheath proximal end 387. Sheath extender 381 may be releasably locked to cannula coupler 364 at a plurality of longitudinal positions using sheath extender lock 386 so as to set an amount of outer cannula 320 that can extend from sheath distal end 388. For example, as depicted in FIGS. 5, 5A, and 5B, sheath extender 381 is locked at a proximal-most position, so that a maximum amount of outer cannula 320 is able to extend from sheath 382. As sheath extender 381 is positioned more distally, less of outer cannula 320 is able to extend from sheath 382.

FIGS. 6, 6A, 6B, 6C, 6D, 6E, 6F, and 6G illustrate anchor needle 400. Anchor needle 400 is another anchor needle adapted to implement the present disclosure's methods of using an anchor needle described above with reference to FIGS. 2A-2E. Anchor needle 400 includes outer cannula 420, anchor cannula 440, cannula support 460, and sheath assembly 480. As will become apparent to those skilled in the art, anchor needle 400 provides series controls for outer cannula 420 and anchor cannula 440, which may result in the benefit that the controls of anchor needle 400 are arranged in a manner relatively similar to those of conventional biopsy needles used in endoscopic ultra-sound fine needle aspiration.

Outer cannula 420 may be generally embodied as, and have the features and functions of, outer cannula 120, outer cannula 120', or outer cannula 220, with certain additions and/or modifications proximal to transverse slot 128 or transverse slot 228 that will be apparent from the present disclosure to those skilled in the art.

Outer cannula 420 extends from outer cannula proximal end 422 to outer cannula distal end 424 and defines outer cannula lumen 426 between outer cannula proximal end 422 and outer cannula distal end 424. Outer cannula lumen 426 is configured to receive therein anchor cannula 440 such that anchor cannula 440 can longitudinally translate within and relative to outer cannula lumen 426. Outer cannula 420 further includes outer cannula proximal section 423 and outer cannula distal section 425. Outer cannula proximal section 423 and outer cannula distal section 425 may for example be formed as a single piece of suitable stainless steel.

Outer cannula 420 also includes cutting handle 428. Cutting handle 428 extends from cutting handle proximal end 432 to cutting handle distal end 429 and defines cutting handle longitudinal cavity 431 between cutting handle proximal end 432 and cutting handle distal end 429. Protrusion 427 extends through longitudinal cavity 431 between cutting handle proximal end 432 and cutting handle distal end 429, and extends partially radially across longitudinal cavity 431, for example as depicted in FIG. 6E. Cutting handle 428 further defines cutting handle lumen 430 between cutting handle proximal end 432 and cutting handle distal end 429. Cutting handle lumen 430 is positioned so it passes longitudinally through protrusion 427, and is sized and shaped to receive outer cannula proximal section 423. Outer cannula proximal section 423 is connected to cutting handle 428 so that longitudinal translation of cutting handle 428 causes corresponding longitudinal translation of outer cannula proximal section 423 and outer cannula distal section 425.

Anchor cannula 440 may be generally embodied as, and have the features and functions of, anchor cannula 140 or anchor cannula 240, with certain additions and/or modifications towards anchor cannula proximal end 142 or anchor cannula proximal end 342 that will be apparent from the present disclosure to those skilled in the art.

Anchor cannula 440 extends from anchor cannula proximal end 442 to anchor cannula distal end 444 and defines anchor cannula lumen 446 between anchor cannula proximal end 442 and anchor cannula distal end 444. Anchor cannula 440 includes anchor cannula proximal section 443 and anchor cannula distal section 445. Anchor cannula proximal section 443 and anchor cannula distal section 445 may in some embodiments be formed from a single piece of stainless steel. Anchor cannula distal section 445 is configured to be received in outer cannula lumen 426 such that anchor cannula distal section 445 can longitudinally translate within and relative to outer cannula lumen 426. For example, anchor cannula distal end 444 can be inserted into outer cannula lumen 426 where outer cannula lumen 426 is exposed at cutting handle proximal end 432, and anchor cannula distal end 444 can then be longitudinally translated distally relative to outer cannula 420 until anchor cannula distal end 444 is positioned within and generally adjacent to outer cannula distal end 424.

Anchor cannula 440 further includes anchor handle 453. Anchor handle 453 extends from anchor handle proximal end 449 to anchor handle distal end 450 and defines anchor handle lumen 451. Anchor cannula proximal section 443 is positioned within and extends through anchor handle lumen 451 such that anchor cannula proximal section proximal edge 452 attaches to anchor handle proximal end 449. Longitudinal translation of anchor handle 453 causes corresponding longitudinal translation of anchor cannula proximal section 443 and anchor cannula distal section 445.

Anchor cannula 440 also includes vacuum connector 447 connected to anchor cannula proximal section 443 and anchor handle proximal end 449. Vacuum connector 447 connects to anchor cannula lumen 446 so that a vacuum can be applied through vacuum connector 447 to anchor cannula lumen 446 and transmitted to anchor cannula distal end 444. Vacuum connector 447 may for example be a Luer connector.

Anchor cannula 440 further includes anchor cannula lock 454 connected to anchor handle 453. Anchor cannula lock 454 is adapted to releasably lock longitudinal translation of anchor cannula 440 relative to cannula support 460. In FIGS. 6, 6A, 6B, 6C, 6D, 6E, 6F, and 6G, anchor cannula lock 454 is depicted as a thumbscrew passing through anchor handle 453 and into anchor handle lumen 451 so as to engage with cannula support 460. The thumbscrew may be turned in a first direction to apply pressure to the exterior surface of cannula support 460 and thereby lock longitudinal translation of anchor cannula 440 relative to cannula support 460. The thumbscrew may be turned in an opposite second direction to remove pressure from the exterior surface of cannula support 460 and thereby unlock anchor cannula 440 relative to cannula support 460 so that anchor cannula 440 is allowed to longitudinally translate relative to cannula support 460. However other suitable mechanisms for releasably locking longitudinal translation of anchor cannula 440 relative to cannula support 460 may be apparent to those skilled in the art. For example, the thumbscrew may be replaced by a push-button locking mechanism or fold-over latch mechanism, each of which works via interlocking teeth or cogs, or by an over-center design which locks due to friction.

Cannula support 460 may be an implementation of, or a form for, cannula support 160.

Cannula support 460 includes inner handle 461. Inner handle 461 extends from inner handle proximal end 462 to inner handle distal end 463. Inner handle 461 includes inner handle proximal section 464 extending from inner handle proximal end 462 to annular stop 465. Inner handle 461 further includes inner handle distal section 466 extending from annular stop 465 to inner handle distal end 463.

Inner handle proximal section 464 includes an inner handle proximal wall 467 that defines inner handle proximal lumen 468 between inner handle proximal end 462 and annular stop 465. Coupling slot 469 extends from inner handle proximal end 462 to annular stop 465, and extends radially through inner handle proximal wall 467 so as to connect inner handle proximal lumen 468 to an exterior of inner handle proximal section 464. Inner handle proximal wall 467 has a shape complementary to cutting handle longitudinal cavity 431, such that cutting handle 428 can be mounted on inner handle proximal section 464 by longitudinally receiving inner handle proximal wall 467 within cutting handle longitudinal cavity 431 so that protrusion 427 is received within coupling slot 469. Cutting handle 428 can thus longitudinally translate along and relative to inner handle proximal section 464. The engagement between protrusion 427 and coupling slot 469 substantially prevents rotation of cutting handle 428 about inner handle proximal section 464 and guides cutting handle 428 along inner handle proximal section 464.

Annular stop 465 and inner handle distal section 466 define inner handle distal lumen 470, which connects with a distal end of inner handle proximal lumen 468. Inner handle distal lumen 470 is sized and shaped to receive outer cannula distal section 425 such that outer cannula distal section 425 can longitudinally translate within and relative to inner handle distal lumen 470. When cutting handle 428 is mounted on inner handle proximal section 464, a proximal portion of outer cannula distal section 425 is contained within inner handle proximal lumen 468 and a distal portion of outer cannula distal section 425 is contained within inner handle distal lumen 470.

Additionally, inner handle proximal section 464 has an exterior diameter that is sized and shaped to be received in and conform to anchor handle lumen 451, such that anchor handle 453 can be mounted on inner handle proximal section 464 to longitudinally translate along and relative to inner handle proximal section 464. For example, with cutting handle 428 mounted on inner handle proximal section 464, anchor handle 453 can then be mounted on inner handle proximal section 464 proximal to cutting handle 428, for example as depicted in FIGS. 6, 6A, and 6C. Anchor cannula distal end 444 can be passed distally through outer cannula lumen 426, with outer cannula 420 itself passed through inner handle proximal lumen 468 and inner handle distal lumen 470, such that both outer cannula 420 and anchor cannula 440 can longitudinally translate relative to cannula support 460 and relative to each other.

Cannula support 460 further includes cutting handle lock 472 connected to inner handle 461 and disposed between cutting handle 428 and annular stop 465. Cutting handle lock 472 may generally include the features and functions of cutting handle lock 372, or suitable alternative mechanisms, with additions and/or modifications that will be apparent from the present disclosure to those skilled in the art. For example, cutting handle lock 472 is adapted to receive and longitudinally translate along inner handle proximal section 464, and to releasably lock to inner handle proximal section 464 at a plurality of longitudinal positions to set a maximum distal longitudinal translation of outer cannula 420 relative to the cannula support 460. Annular stop 465 is sized and shaped to engage with a distal edge of cutting handle lock 472 to prevent cutting handle lock 472 from translating distal to annular stop 465. Cutting handle 428 (and thus outer cannula 420) may thus be longitudinally translated distally relative to cannula support 460 to an extent determined by the position of cutting handle lock 472 and by whether cutting handle lock 472 is in a locked or unlocked configuration.

Anchor needle 400 can be operated to together introduce outer cannula distal end 424 and anchor cannula distal end 444 to a target lesion, or withdraw them from a target lesion, while keeping the transverse slot 499 of outer cannula 420 internally covered by anchor cannula 440, for example in order to implement aspects of the method described with reference to FIGS. 2A-2E. Cutting handle proximal end 432 and anchor handle distal end 450 are configured to engage with each other such that, with anchor handle 453 abutting cutting handle 428, and anchor cannula lock 454 in its unlocked configuration, when anchor cannula 440 is longitudinally translated distally relative to cannula support 460 (e.g. due to distal longitudinal translation of anchor handle 453), outer cannula 420 and anchor cannula 440 are substantially prevented from longitudinally translating relative to each other. Similarly, with anchor handle 453 abutting cutting handle 428, and anchor cannula lock 454 in its unlocked configuration, when outer cannula 420 is longitudinally translated proximally relative to cannula support 460 (e.g. due to proximal longitudinal translation of cutting handle 428), outer cannula 420 and anchor cannula 440 are substantially prevented from longitudinally translating relative to each other. Thus when anchor cannula lock 454 is unlocked, distal longitudinal translation of anchor cannula 440 with anchor handle 453 abutting cutting handle 428 causes corresponding distal longitudinal translation of outer cannula 420, for example in order to introduce outer cannula distal end 424 and anchor cannula distal end 444 together into a target lesion with the transverse slot 499 of outer cannula 420 blocked by anchor cannula 440. Similarly, when anchor cannula lock 454 is unlocked, proximal longitudinal translation of outer cannula 420 with anchor handle 453 abutting cutting handle 428 causes corresponding proximal longitudinal translation of anchor cannula 440, for example in order to withdraw outer cannula distal end 424 and anchor cannula distal end 444 together from a target lesion with the transverse slot 499 of outer cannula 420 blocked by anchor cannula 440.

Additionally, anchor needle 400 can be operated to reciprocate outer cannula 420 so as to alternately anchor the anchor cannula 440 to target tissue and then cut a sample from the anchored target tissue, for example in order to implement aspects of the method described with reference to FIGS. 2A-2E. When anchor handle 453 and cutting handle lock 472 are sufficiently spaced apart that cutting handle 428 has clearance to longitudinally translate between them, outer cannula 420 is able to longitudinally translate relative to both anchor cannula 440 and cannula support 460 for a distance substantially equivalent to that clearance. For example, if (a) anchor cannula lock 454 is locked to inner handle 461 at inner handle proximal end 462, (b) cutting handle lock 472 is locked to inner handle 461 at a position abutting annular stop 465, and (c) cutting handle proximal end 432 is abutting anchor handle distal end 450, then outer cannula 420 is able to longitudinally translate distally for the distance between cutting handle distal end 429 and the proximal end of cutting handle lock 472. Similarly, if (a) anchor cannula lock 454 is locked to inner handle 461 at inner handle proximal end 462, (b) cutting handle lock 472 is locked to inner handle 461 at a position abutting annular stop 465, and (c) cutting handle distal end 429 abutting the proximal end of cutting handle lock 472, then outer cannula 420 is able to longitudinally translate proximally for the distance between cutting handle proximal end 432 and anchor handle distal end 450. This may for example allow outer cannula 420 to alternately (1) uncover the transverse slot 499 of outer cannula 420 so anchor cannula distal end 444 can anchor tissue, and (2) cover the transverse slot 499 of outer cannula 420 to cut a tissue sample.

Moreover, anchor needle 400 can be operated to retrieve a tissue sample in anchor cannula lumen 446, for example in order to implement aspects of the method described with reference to FIGS. 2A-2E. When anchor cannula lock 454 is unlocked, anchor cannula 440 can be completely withdrawn proximally from outer cannula 420 and cannula support 460, for example if the user holds cutting handle 428 in position relative to cannula support 460 during the withdrawal. This may for example allow the user to retrieve a tissue sample in anchor cannula lumen 446 from anchor cannula distal end 444. Alternatively, in embodiments in which anchor needle 400 includes a closed needle tip that can transition between a closed configuration and an open configuration, outer cannula 420 and anchor cannula 440 could together be completely withdrawn from cannula support 460, and then anchor cannula 440 could be translated distally relative to outer cannula 420 such that anchor cannula distal end 444 is exposed through outer cannula distal end 424.

Those skilled in the art will recognize how the features and functions of outer cannula 420, anchor cannula 440, and cannula support 460 can be readily used to implement the method of operating an anchor needle to remove a tissue sample from a target lesion described with reference to FIGS. 2A-2E.

Anchor needle 400 further includes sheath assembly 480. Sheath assembly 480 includes sheath extender 481, sheath 482, and sheath extender lock 486. Sheath assembly 480, sheath extender 481, sheath 482, and sheath extender lock 486 may generally include the same respective features and functions of sheath assembly 380, sheath extender 381, sheath 382, and sheath extender lock 386, with additions and/or modifications that will be apparent from the present disclosure to those skilled in the art. In sheath extender 481, sheath extender lumen 485 is sized and adapted to receive and conform to an exterior surface of inner handle distal section 466, such that sheath extender 481 can longitudinally translate on and relative to inner handle distal section 466.

Sheath extender 481 may be releasably locked to inner handle distal section 466 at a plurality of longitudinal positions using sheath extender lock 486 so as to set an amount of outer cannula 420 that can extend from sheath distal end 488.

Anchor needle 400 is depicted in FIG. 6 as being connected to vacuum source 500. Vacuum source 500 includes negative pressure generator 501 and valve 502. In Fig. 6, negative pressure generator 501 is depicted as a syringe, but other negative pressure generators could be used, including for example vacuum tanks, compressors, etc. In Fig. 6, valve 502 is depicted as a stopcock, but other valves could be used to alternately allow a vacuum from pressure generator 501 to be applied to anchor cannula lumen 446 and not applied to anchor cannula lumen 446. When valve 502 is a stopcock, one Luer connector of the stopcock could be connected to a Luer connector of pressure generator 501 and the other Luer connector of the stopcock could be connected to a Luer connector of vacuum connector 447.

Those of skill in the art will appreciate that embodiments not expressly illustrated herein may be practiced within the scope of the claims, including that features described herein for different embodiments may be combined with each other and/or with currently-known or future-developed technologies while remaining within the scope of the claims. Although specific terms are employed herein, they are used in a generic and descriptive sense only and not for purposes of limitation unless specifically defined by context, usage, or other explicit designation. It is therefore intended that the foregoing detailed description be regarded as illustrative rather than limiting. And, it should be understood that the following claims, including all equivalents, are intended to define the spirit and scope of this invention. Furthermore, the advantages described above are not necessarily the only advantages of the invention, and it is not necessarily expected that all of the described advantages will be achieved with every embodiment. In the event of any inconsistent disclosure or definition from the present application conflicting with any document incorporated by reference, the disclosure or definition herein shall be deemed to prevail.

## Claims

1. An anchor needle comprising:
an anchor cannula defining an anchor cannula lumen and comprising an anchor cannula distal end, the anchor cannula lumen adapted to transmit a vacuum to the anchor cannula distal end;
an outer cannula defining an outer cannula lumen adapted to receive the anchor cannula, the outer cannula comprising an outer cannula proximal end, an outer cannula distal end, and a transverse slot disposed between the outer cannula proximal end and the outer cannula distal end;
a cannula support adapted to hold the anchor cannula and the outer cannula such that the anchor cannula and the outer cannula are able to independently longitudinally translate relative to the cannula support; and
an anchor cannula lock adapted to releasably lock longitudinal translation of the anchor cannula relative to the cannula support;
wherein the anchor needle is adapted to be arranged in a tissue-anchoring configuration in which:
the anchor cannula lock is arranged in a locked anchor cannula configuration that prevents the anchor cannula from longitudinally translating relative to the cannula support; and
the outer cannula is longitudinally positioned relative to the anchor cannula so that the anchor cannula allows tissue to be drawn through the transverse slot by the vacuum.

2. The anchor needle of claim 1, wherein the anchor cannula lock is adapted to be arranged in an unlocked anchor cannula configuration that allows the anchor cannula to longitudinally translate relative to the cannula support.

3. The anchor needle according to claim 1 or 2, wherein when the anchor needle is arranged in the tissue-anchoring configuration, the anchor cannula distal end is positioned such that when the tissue is drawn through the transverse slot by the vacuum, the tissue will be anchored to the anchor cannula by the vacuum.

4. The anchor needle according to claim 3, wherein when the anchor needle is arranged in the tissue-anchoring configuration, the tissue will be anchored to the anchor cannula by being drawn at least partially into the anchor cannula lumen by the vacuum.

5. The anchor needle according to any of claims 1-4, wherein arranging the locked anchor cannula in the locked anchor cannula configuration does not prevent the outer cannula from longitudinally translating relative to the cannula support and the anchor cannula.

6. The anchor needle according to claim 5, wherein when the tissue enters the transverse slot, a tissue sample will be cut by longitudinally translating the outer cannula relative to the anchor cannula.

7. The anchor needle according to any of claims 1-6, wherein the outer cannula distal end comprises a closed needle tip.

8. The anchor needle according to claim 7, wherein the closed needle tip includes a folded portion of a top of the outer cannula that is angled towards a portion of a bottom of the outer cannula and that closes off the outer cannula lumen distally.

9. The anchor needle according to claim 8, wherein the portion of the top of the outer cannula is able to move from a closed configuration contacting the portion of the bottom of the outer cannula to an open configuration separated from the portion of the bottom of the outer cannula, such that the anchor cannula distal end is able to move distally through the closed needle tip to transition the portion of the top of the outer cannula from the closed configuration to the open configuration.

10. The anchor needle according to any of claims 1-9, further comprising a handle lock adapted to longitudinally translate along the cannula support and releasably lock to the cannula support at a plurality of longitudinal positions to set a maximum distal longitudinal translation of the outer cannula relative to the cannula support.

11. The anchor needle according to claim 10, further comprising a cutting handle attached to the outer cannula proximal end such that longitudinal translation of the cutting handle causes corresponding longitudinal translation of the outer cannula, wherein the handle lock, when locked to the cannula support, is configured to engage with the cutting handle to set a maximum longitudinal distal longitudinal translation of the outer cannula relative to the cannula support.

12. The anchor needle according to any of claims 1-11, wherein the cannula support comprises a Y-shaped structure, the Y-shaped structure comprising:
an anchor cannula holder defining an anchor cannula holder lumen adapted to receive the anchor cannula;
an outer cannula holder defining an outer cannula holder lumen adapted to receive the outer cannula; and
a cannula coupler defining a cannula coupler lumen adapted to receive the outer cannula and the anchor cannula.

13. The anchor needle according to claim 12, wherein the outer cannula comprises an outer cannula proximal section, an outer cannula distal section, and an opening in the outer cannula proximal section, the opening connecting with the outer cannula lumen and adapted so that the anchor cannula can pass through the opening into the outer cannula lumen when the outer cannula and the anchor cannula are received by the Y-shaped structure.

14. The anchor needle according to any of claims 1-11, further comprising a cutting handle attached to an outer cannula proximal end, wherein the cannula support comprises an inner handle, the inner handle comprising an inner handle lumen and a coupling slot extending radially to connect with the inner handle lumen, and wherein the cutting handle comprises a protrusion configured to engage with the coupling slot so the cutting handle can longitudinally translate relative to the inner handle.

15. The anchor needle according to claim 14, further comprising an anchor handle attached to an anchor cannula proximal end, the anchor handle comprising an anchor handle lumen configured to receive the inner handle and longitudinally translate relative to the inner handle.
